# EUROPÄISCHE PATENTSCHRIFT

(11) **EP 3 128 967 B1**
(45) Veröffentlichungstag und Bekanntmachung des Hinweises auf die Patenterteilung: **26.12.2018**
(21) Anmeldenummer: 16709262.6
(22) Anmeldetag: 02.02.2016
(51) Int. Cl.: A61F 9/007

(54) **HOHLNADEL FÜR EIN AUGENCHIRURGISCHES INSTRUMENT**
HOLLOW NEEDLE FOR AN OPHTHALMIC SURGICAL INSTRUMENT
AIGUILLE CREUSE POUR UN INSTRUMENT DE CHIRURGIE OCULAIRE

(30) Priorität: 20.04.2015 DE 102015207150
(43) Veröffentlichungstag der Anmeldung: 15.02.2017
(73) Patentinhaber: Geuder AG, 69126 Heidelberg (DE)
(72) Erfinder: WOLSCHENDORF, Marc, 69198 Schriesheim (DE); ENGEL, Stefan, 69124 Heidelberg (DE); FRAUENFELD, Dieter, 69121 Heidelberg (DE); DRAHEIM, René, 69207 Sandhausen (DE); GEUDER, Volker, 69126 Heidelberg (DE)
(74) Vertreter: Patent- und Rechtsanwälte Ullrich & Naumann
(86) Internationale Anmeldenummer: PCT/DE2016/200063
(87) Internationale Veröffentlichungsnummer: WO 2016/169556

(56) Entgegenhaltungen:
- EP-A1- 2 737 884
- DE-C1- 19 646 881
- JP-A- 2009 095 662
- US-A1- 2009 099 536

## Beschreibung

Die Erfindung betrifft eine Hohlnadel für ein augenchirurgisches Instrument zur in-vivo-Zertrümmerung organischer Linsen mittels Ultraschall, mit einem am proximalen Ende ausgebildeten Anschlussbereich zum Ankoppeln an das Instrument und einem am distalen Ende ausgebildeten Nadelkopf mit einer Wirkfläche zum Abstrahlen von Ultraschallwellen, wobei sich in Axialrichtung ein Absaugkanal zum Absaugen von Linsentrümmern durch die Hohlnadel hindurch erstreckt, dessen Öffnung durch die Wirkfläche begrenzt ist, wobei der Absaugkanal im Nadelkopf zumindest bereichsweise in Richtung des proximalen Endes konisch zulaufend ausgebildet ist und wobei über diesen Bereich hinweg in Axialrichtung verlaufende Einschnitte in der Wandung des Absaugkanals ausgebildet sind.
Hohlnadeln der in Rede stehenden Art sind seit Jahren aus der Praxis bekannt. Eine entsprechende Hohlnadel ist in der US 6,074,396 A gezeigt. Dabei ist der Absaugkanal innerhalb des Nadelkopfes gestuft ausgebildet, wobei zumindest einige dieser Stufen prismenförmige Einschnitte aufweisen, um die Zertrümmerung des Linsenkörpers zu verbessern.
Des Weiteren zeigt die DE 196 46 881 C1 eine Hohlnadel zur Anwendung in der Ophthalmologie. Im Konkreten handelt es sich dabei um eine Hohlnadel für ein augenchirurgisches Instrument zur in-vivo-Zertrümmerung von Linsen durch Hochfrequenzbetätigung der Hohlnadel, wobei die Hohlnadel gleichzeitig zum Absaugen von Linsen durch einen inneren Absaugkanal dient. Die Hohlnadel umfasst ein ringförmig ausgebildetes Stirnende, das die Öffnung des Absaugkanals bildet.
Das Dokument DE 196 28 252 A1 zeigt eine Phakoemulsifikationsnadel mit einem am distalen Ende ausgebildeten Nadelkopf, der eine Wirkfläche zum Abstrahlen von Ultraschallwellen aufweist. Des Weiteren erstreckt sich ein Absaugkanal durch die Nadel hindurch, wobei der Absaugkanal im Nadelkopf konisch zulaufend ausgebildet ist und in Axialrichtung verlaufende Einschnitte in seiner Wandung aufweist.

Aus der EP 2 737 884 A1 ist eine Phakoemulsifikationsnadel vorbekannt, die an ihrem distalen Ende einen Vorsprung aufweist. Des Weiteren sind in den Dokumenten DE 196 46 881 C1, JP 2009 095 662 A, US 2009/0099536 A1, US 2012/01972151 A1 und US 2014/0243842 A1 unterschiedlich ausgebildete Phakoemulsifikationsnadeln offenbart.

Ultraschallbetätigte Hohlnadeln der gattungsbildenden Art werden bei Kataraktoperationen in der Augenchirurgie verwendet. Das freie Ende der Hohlnadel wird in eine hochfrequente Bewegung versetzt und unmittelbar an den Katarakt herangeführt. Vom ringförmigen Stirnende werden Ultraschallwellen zur Emulsifikation des Gewebes abgestrahlt. Abgetrennte Linsenteile bzw. Linsentrümmer werden durch die Hohlnadel hindurch gemeinsam mit einer dem Auge zugeführten Spülflüssigkeit abgeführt.

Zur Verstärkung des emittierten Ultraschallfeldes ist es bereits bekannt, das stirnseitige bzw. distale Ende der Hohlnadel, d.h. die dortige Wirkfläche, gezahnt auszugestalten. Die zum Abstrahlen von Ultraschallwellen dienende Wirkfläche wird dadurch vergrößert, so dass die Effizienz des Instruments bzw. der Hohlnadel verbessert ist.

Die bekannten Hohlnadeln sind in der Praxis jedoch problematisch, da die Zertrümmerung der Linsen nur sehr langsam erfolgt. Um die Belastung durch den Eingriff für den Patienten möglichst gering zu halten, ist man jedoch bemüht, die Dauer des Eingriffs zu minimieren. Des Weiteren ist problematisch, dass bei der gattungsbildenden Hohlnadel mitunter größere Linsentrümmer abgesaugt werden, die sodann den Absaugkanal verstopfen und in diesem nur sehr langsam weiter zerkleinert werden.

Der vorliegenden Erfindung liegt daher die Aufgabe zugrunde, eine Hohlnadel für ein augenchirurgisches Instrument der gattungsbildenden Art derart auszugestalten und weiterzubilden, dass mit konstruktiv einfachen Mitteln in kurzer Zeit eine effektive Zertrümmerung organischer Linsen ermöglicht ist.

Erfindungsgemäß wird die voranstehende Aufgabe durch die Merkmale des Anspruches 1 gelöst.

In erfindungsgemäßer Weise ist dabei zunächst erkannt worden, dass die zugrundeliegende Aufgabe durch eine geschickte Ausgestaltung der Geometrie des Absaugkanals im Nadelkopf gelöst werden kann. Dazu ist der Absaugkanal im Nadelkopf zumindest bereichsweise in Richtung des proximalen Endes konisch zulaufend ausgebildet, so dass sich der Durchmesser des Absaugkanals im Nadelkopf in Richtung des proximalen Endes verringert. Somit sind innerhalb des Absaugkanals keine radial verlaufenden Stufen bzw. Kanten vorhanden, an denen Linsentrümmer hängen bleiben und somit den Absaugkanal verstopfen. In weiter erfindungsgemäßer Weise ist erkannt worden, dass die Zertrümmerung der Linsen in verblüffend einfacher Weise verbessert werden kann, indem die Wandung des Absaugkanals über den gesamten konisch zulaufenden Bereich hinweg axial verlaufende Einschnitte aufweist. Durch diese einfache konstruktive Maßnahme ist die Abstrahlung der Ultraschallwellen optimiert, so dass eine verbesserte Zertrümmerung organischer Linsen möglich ist. Dabei wird durch die konische Ausgestaltung des Absaugkanals und die in dem konischen Bereich angeordneten Einschnitte ein kombinatorischer Effekt realisiert, der zu einer besonders schnellen und sicheren Zertrümmerung des Linsengewebes führt.

Zweckmäßigerweise können unmittelbar am distalen Ende des Nadelkopfes in der Wandung des Absaugkanals, insbesondere tetraederförmige, Ausnehmungen ausgebildet sein. Dabei können die Ausnehmungen in Umfangsrichtung, beispielsweise radialsymmetrisch, angeordnet sein. Der Durchmesser des Absaugkanals kann in diesem Bereich zusätzlich vergrößert sein. Diese Ausgestaltung bietet den Vorteil, dass unmittelbar am distalen Ende der Hohlnadel die Abstrahlung der Ultraschallwellen maximiert ist, was die Effektivität der Zertrümmerung verbessert.

Im Konkreten können die Einschnitte einen mehreckigen Querschnitt aufweisen. Beispielsweise können die Einschnitte einen dreieckigen Querschnitt aufweisen, so dass diese besonders einfach herstellbar sind. Durch diese konstruktive Maßnahme ist zwischen den Einschnitten eine zahnartige Struktur in der Wandung des Absaugkanals realisiert, die sich zur Zerkleinerung besonders harter Linsenteile eignet.

Je nach Härte der zu zertrümmernden Linse können die Einschnitte in Umfangsrichtung radialsymmetrisch oder asymmetrisch angeordnet sein.

In vorteilhafter Weise kann sich der konisch zulaufende Bereich durch einen Teil des Nadelkopfes hindurch erstrecken. Des Weiteren ist denkbar, dass sich der konisch zulaufende Bereich durch den gesamten Nadelkopf erstreckt, so dass eine maximale Fläche zur Abstrahlung von Ultraschallwellen realisiert ist.

In weiter vorteilhafter Weise kann der konisch zulaufende Bereich unmittelbar am distalen Ende der Hohlnadel beginnen, so dass eingesaugte Linsentrümmer bereits zu Beginn des Absaugkanals mit einem Maximum an Ultraschallwellen beaufschlagt werden. Sofern am distalen Ende des Absaugkanals Ausnehmungen vorgesehen sind, kann der konisch zulaufende Bereich unmittelbar nach den Ausnehmungen beginnen. Des Weiteren ist denkbar, dass der konisch zulaufende Bereich versetzt vom distalen Ende, d.h. in Richtung des proximalen Endes, beginnt. Eine solche Konstruktion eignet sich zur besonders schonenden Zerstörung der Linse.

Um den sog. "jack hammer-Anteil", d.h. die Effektivität der Hohlnadel, weiter zu erhöhen, können die Einschnitte in Richtung des proximalen Endes über den konisch zulaufenden Bereich hinaus verlaufen. Zur Verstärkung dieses Effektes ist des Weiteren denkbar, dass die äußere Wandung des Nadelkopfes vieleckig, beispielsweise vier-, fünf-, sechs- oder achteckig, ausgebildet ist. Zur Schonung des Gewebes, das um die zu zertrümmernde Linse liegt, kann die äußere Wandung des Nadelkopfes rund ausgebildet sein.

Um die Wirkfläche zu vergrößern, kann diese schräg zu der Axialrichtung verlaufen. Des Weiteren ist denkbar, dass die Wirkfläche senkrecht zu der Axialrichtung verläuft, um somit das umliegende Gewebe vor Verletzungen zu schützen.

In weiter erfindungsgemäßer Weise weist die Wirkfläche zwei in unterschiedlichen Winkeln gegenüber der Axialrichtung verlaufende Wirkflächenbereiche auf, so dass die Wirkfläche in ihrer Gesamtheit schnabelartig ausgebildet ist. Durch die schnabelartige Geometrie ist eine äußerst effektive und konzentrierte Abstrahlung von Ultraschallwellen möglich, so dass auch harte Bereiche der zu zertrümmernden Linse, beispielsweise ein bereits längere Zeit existierender Katarakt, zügig zerstört werden.

In weiter vorteilhafter Weise ist die Wirkfläche durch eine äußere Kante begrenzt, wobei die äußere Kante angefast, stumpf oder scharf ausgebildet sein kann. Eine angefaste bzw. stumpfe Ausbildung der Kante dient zur Vermeidung von Verletzungen des umliegenden Gewebes. Durch eine scharfe Ausgestaltung der äußeren Kante ist eine besonders effektive Zertrümmerung der Linse möglich.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein erstes Ausführungsbeispiel einer Hohlnadel,
- Fig. 2: in einer schematischen Ansicht ein weiteres Ausführungsbeispiel einer Hohlnadel,
- Fig. 3: in einer schematischen, frontalen Ansicht die Hohlnadel gemäß Fig. 1,
- Fig. 4: in einer schematischen, frontalen Ansicht die Hohlnadel gemäß Fig. 2,
- Fig. 5: in einer schematischen Ansicht, vergrößert, ein Ausführungsbeispiel des Nadelkopfes einer Hohlnadel,
- Fig. 6: in einer schematischen Ansicht, vergrößert, den Nadelkopf der Hohlnadel gemäß Fig. 4,
- Fig. 7: in einer geschnittenen Ansicht, vergrößert, ein weiteres Ausführungsbeispiel des Nadelkopfes einer Hohlnadel,
- Fig. 8: in einer schematischen Ansicht, vergrößert, ein weiteres Ausführungsbeispiel des Nadelkopfes einer Hohlnadel,
- Fig. 9: in einer schematischen Ansicht, vergrößert, ein weiteres Ausführungsbeispiel des Nadelkopfes einer Hohlnadel,
- Fig. 10: in einer schematischen Ansicht, ein Ausführungsbeispiel des Nadelkopfes einer erfindungsgemäßen Hohlnadel und
- Fig. 11: in einer schematischen Ansicht, vergrößert, ein weiteres Ausführungsbeispiel des Nadelkopfes einer erfindungsgemäßen Hohlnadel.

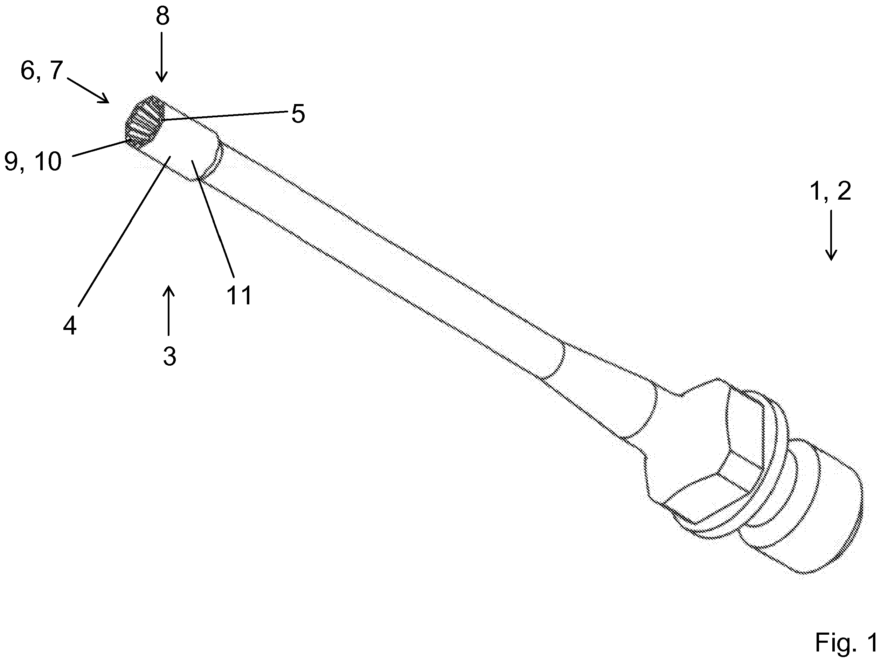

Figur 1 zeigt in einer schematischen Ansicht ein erstes Ausführungsbeispiel einer Hohlnadel für ein augenchirurgisches Instrument zur in-vivo-Zertrümmerung organischer Linsen mittels Ultraschall. Die Hohlnadel umfasst einen am proximalen Ende 1 ausgebildeten Anschlussbereich 2 zum Ankoppeln an das in den Figuren nicht gezeigte Instrument und einen am distalen Ende 3 ausgebildeten Nadelkopf 4. Der Nadelkopf 4 weist eine Wirkfläche 5 zum Abstrahlen von Ultraschallwellen auf. Durch die Hohlnadel hindurch erstreckt sich in Axialrichtung ein Absaugkanal 6 zum Absaugen von Linsentrümmern, dessen Öffnung 7 durch die Wirkfläche 5 begrenzt ist. Der Absaugkanal 6 verläuft in diesem Ausführungsbeispiel koaxial zu der Symmetrieachse der Hohlnadel.

Der Absaugkanal 6 ist im Nadelkopf 4 zumindest bereichsweise in Richtung des proximalen Endes 1 konisch zulaufend ausgebildet. Über den gesamten konisch zulaufenden Bereich 8 hinweg sind in Axialrichtung verlaufende Einschnitte 9 in der Wandung 10 des Absaugkanals 6 ausgebildet. Die Einschnitte 9 sind in Umfangsrichtung radialsymmetrisch angeordnet. Zur besseren Übersichtlichkeit ist in den Figuren jeweils lediglich ein Einschnitt 9 mit einem Bezugszeichen versehen.

Die äußere Wandung 11 des Nadelkopfes ist in dem hier dargestellten Ausführungsbeispiel achteckig ausgebildet, wodurch sich der "jack hammer-Anteil" bei der Zertrümmerung der Linsen erhöht.

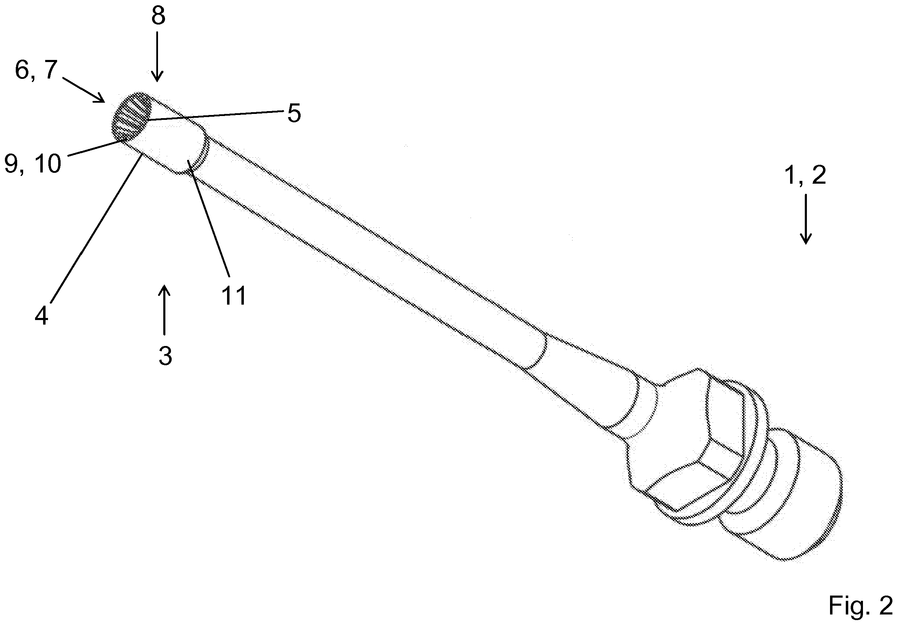

Figur 2 zeigt in einer schematischen Ansicht ein weiteres Ausführungsbeispiel einer Hohlnadel. Das Ausführungsbeispiel gemäß Figur 2 entspricht dem Ausführungsbeispiel gemäß Figur 1, wobei die äußere Wandung 11 des Nadelkopfes 4 in Figur 2 rund ausgebildet ist. Die in Figur 2 dargestellte Hohlnadel eignet sich somit zur besonders gewebeschonenden Zertrümmerung von Linsen.

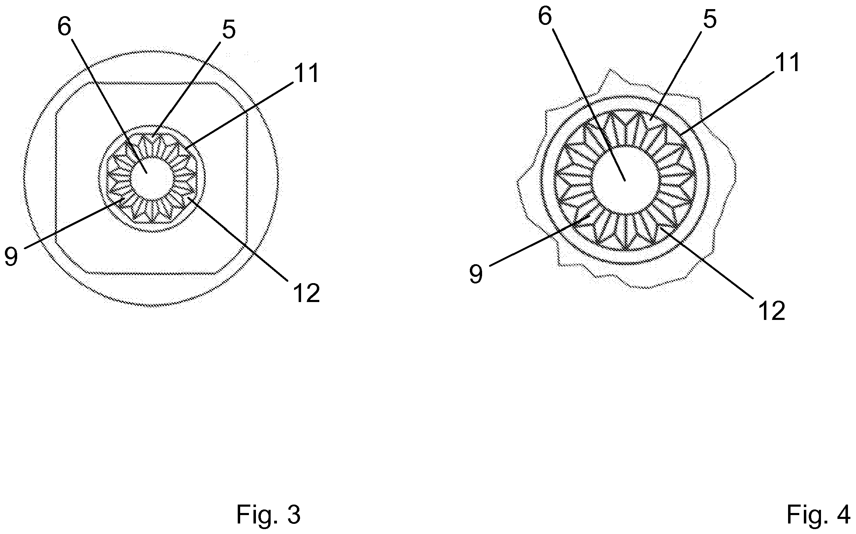

Figur 3 zeigt in einer schematischen, frontalen Ansicht die Hohlnadel gemäß Figur 1. Dabei ist nochmals deutlich die achteckige äußere Wandung 11 des Nadelkopfes 4 erkennbar. Des Weiteren geht aus Figur 3 eindeutig hervor, dass der Absaugkanal 6 im Nadelkopf 4 in Richtung des proximalen Endes 1 konisch zulaufend ausgebildet ist und in Axialrichtung verlaufende Einschnitte 9 aufweist. Der Absaugkanal 6 verläuft koaxial zu Symmetrieachse der Hohlnadel. Die Einschnitte 9 weisen einen dreieckigen Querschnitt auf, so dass zwischen den Einschnitten Zähne 12 in der Wandung 10 realisiert sind. Zur Verbesserung der Übersichtlichkeit sind in den Figuren jeweils nur einzelne Zähne 12 mit Bezugszeichen versehen.

Figur 4 zeigt in einer schematischen, frontalen Ansicht die Hohlnadel gemäß Figur 2. Dabei ist nochmals deutlich zu erkennen, dass die äußere Wandung 11 rund ausgebildet ist. Des Weiteren entspricht das Ausführungsbeispiel gemäß Figur 4 den in den Figuren 1 und 3 dargestellten Ausführungsbeispielen.

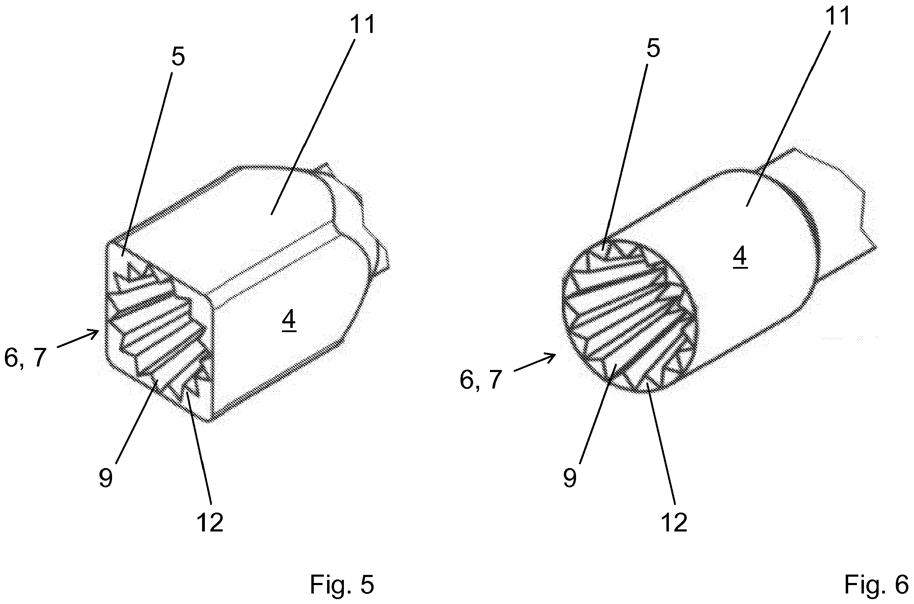

Figur 5 zeigt in einer schematischen Ansicht, vergrößert, ein Ausführungsbeispiel des Nadelkopfes 4 einer erfindungsgemäßen Hohlnadel. Dabei ist die äußere Wandung 11 viereckig ausgebildet. Des Weiteren entspricht Figur 5 dem in den Figuren 1 und 3 dargestellten Ausführungsbeispiel, so dass sich weitere Ausführungen hierzu erübrigen.

Figur 6 zeigt in einer schematischen Ansicht, vergrößert, den Nadelkopf 4 der Hohlnadel gemäß Figur 4. In dieser Darstellung ist nochmals der dreieckige Querschnitt der Einschnitte 9 zu erkennen, durch welchen die Zähne 12 realisiert werden.

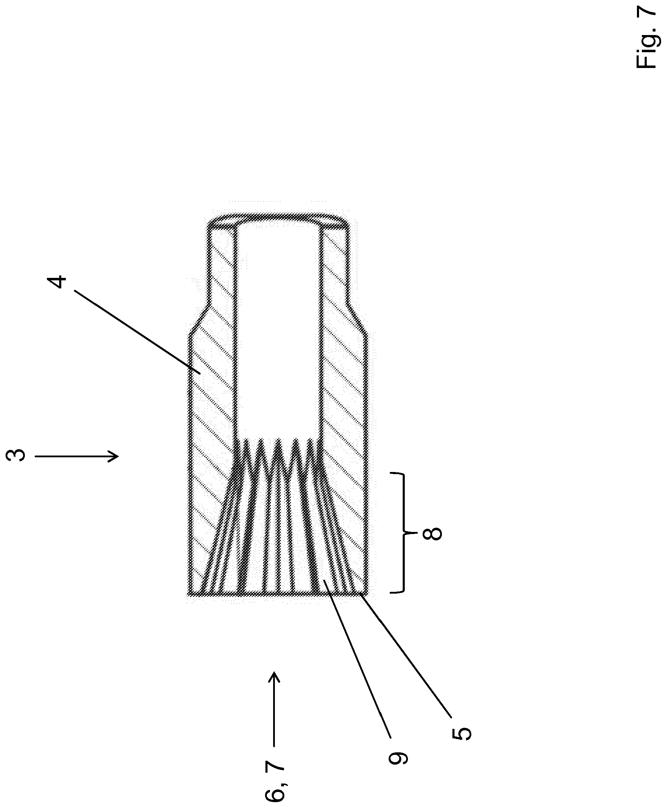

Figur 7 zeigt in einer geschnittenen Ansicht, vergrößert, ein weiteres Ausführungsbeispiel des Nadelkopfes 4 einer erfindungsgemäßen Hohlnadel. Bei dem in Figur 7 gezeigten Ausführungsbeispiel beginnt der konisch zulaufende Bereich 8 unmittelbar am distalen Ende 3 des Nadelkopfes 4. Der Bereich 8 erstreckt sich nicht über den gesamten Nadelkopf. Die Einschnitte 9 verlaufen in Richtung des proximalen Endes 1 über den konisch zulaufenden Bereich 8 hinaus.

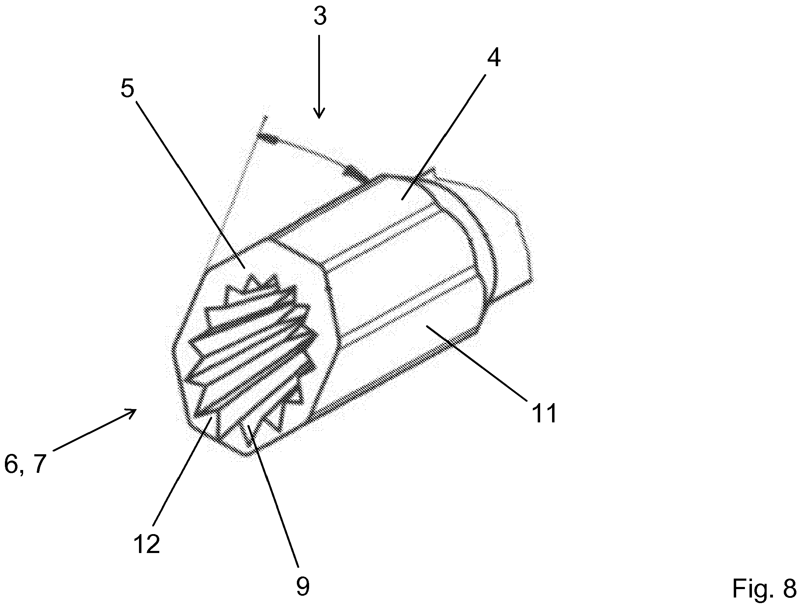

Figur 8 zeigt in einer geschnittenen Ansicht, vergrößert, ein weiteres Ausführungsbeispiel des Nadelkopfes 4 einer Hohlnadel. Die äußere Wandung 11 des Nadelkopfes 4 ist achteckig ausgebildet. Des Weiteren verläuft die Wirkfläche 5 schräg zu der Axialrichtung, so dass die Wirkfläche 5 vergrößert ist. Weiterhin ist dargestellt, dass der Absaugkanal 6 nicht koaxial, sondern parallel versetzt zu der Symmetrieachse der Hohlnadel verläuft.

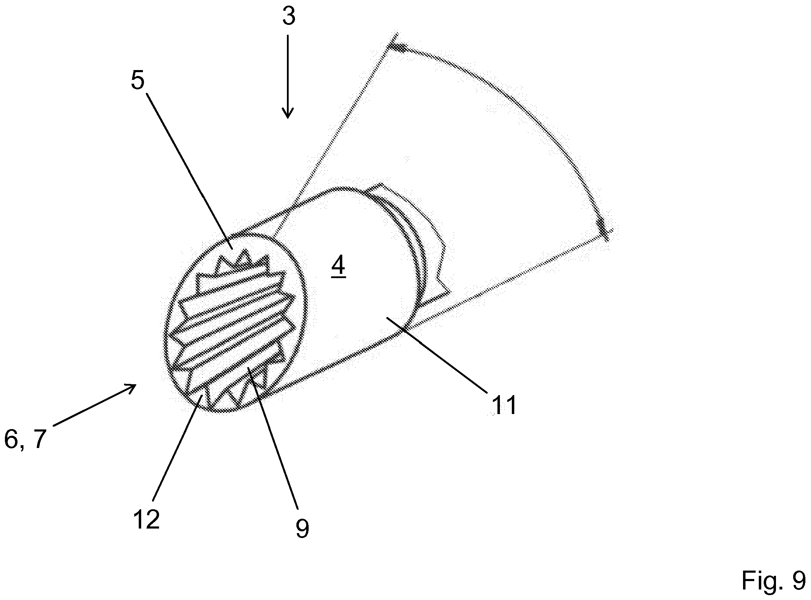

Figur 9 zeigt in einer geschnittenen Ansicht, vergrößert, ein weiteres Ausführungsbeispiel des Nadelkopfes 4 einer erfindungsgemäßen Hohlnadel. Der Nadelkopf 4 weist eine äußere Wandung 11 auf, die rund ausgestaltet ist. Dabei ist die Wirkfläche 5 schräg zu der Axialrichtung verlaufend. Auch hier verläuft der Absaugkanal 6 nicht koaxial, sondern parallel versetzt zu der Symmetrieachse der Hohlnadel.

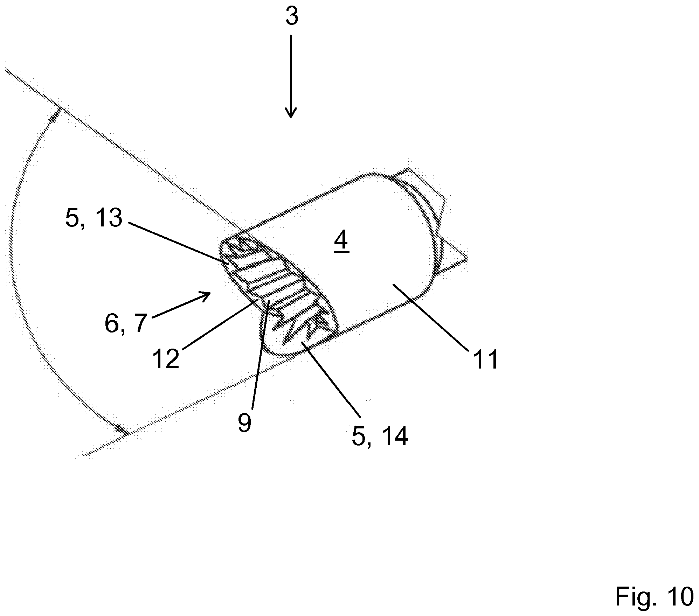

Figur 10 zeigt in einer geschnittenen Ansicht, vergrößert, ein Ausführungsbeispiel des Nadelkopfes 4 einer erfindungsgemäßen Hohlnadel. Die Wirkfläche 5 des Nadelkopfes 4 umfasst in diesem Ausführungsbeispiel zwei Wirkflächenbereiche 13, 14. Der erste Wirkflächenbereich 13 verläuft gegenüber der Axialrichtung in einem anderen Winkel als der zweite Wirkflächenbereich 14, so dass die Wirkfläche 5 schnabelartig ausgebildet ist. Bei einer solchen Konstruktion ist der "jack hammer-Anteil" besonders groß, was zu einer effektiven Zertrümmerung der Linsen führt. Die äußere Wandung 11 ist rund ausgebildet. Dabei ist jedoch auch denkbar, dass die äußere Wandung 11 vieleckig, beispielsweise vier- oder achteckig, ausgestaltet ist.

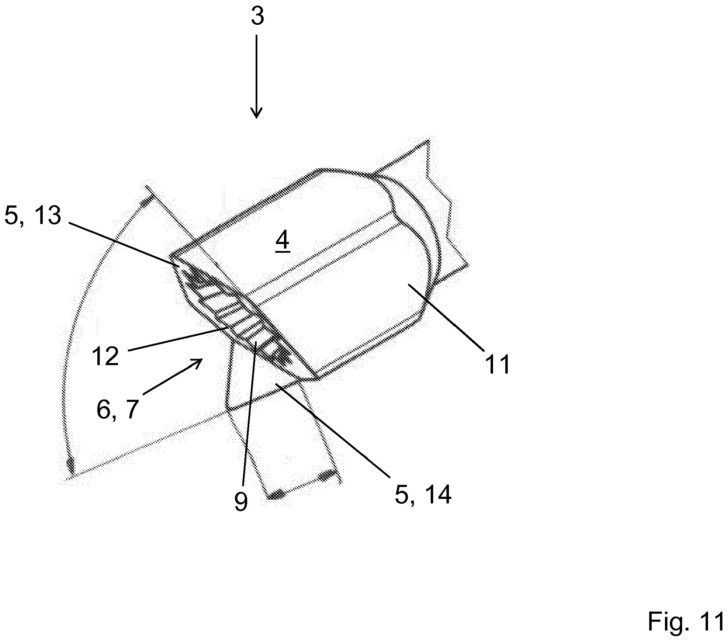

Figur 11 in einer geschnittenen Ansicht, vergrößert, ein weiteres Ausführungsbeispiel des Nadelkopfes 4 einer erfindungsgemäßen Hohlnadel. Die Wirkfläche 5 des Nadelkopfes 4 umfasst in diesem Ausführungsbeispiel zwei Wirkflächenbereiche 13, 14. Der erste Wirkflächenbereich 13 verläuft gegenüber der Axialrichtung in einem anderen Winkel als der zweite Wirkflächenbereich 14, so dass die Wirkfläche 5 schnabelartig ausgebildet ist. Weiterhin geht aus Figur 11 hervor, dass der zweite Wirkflächenbereich 14 in Radialrichtung derart weit außen angeordnet ist, dass sich der Absaugkanal 6 nicht bis hin zu dem zweiten Wirkflächenbereich 14 hin erstreckt.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: proximales Ende
- 2: Anschlussbereich
- 3: distales Ende
- 4: Nadelkopf
- 5: Wirkfläche
- 6: Absaugkanal
- 7: Öffnung
- 8: konischer Bereich
- 9: Einschnitte
- 10: Wandung (Absaugkanal)
- 11: äußere Wandung
- 12: Zähne
- 13: erster Wirkflächenbereich
- 14: zweiter Wirkflächenbereich

## Patentansprüche

1. Hohlnadel für ein augenchirurgisches Instrument zur in-vivo-Zertrümmerung organischer Linsen mittels Ultraschall, mit einem am proximalen Ende (1) ausgebildeten Anschlussbereich (2) zum Ankoppeln an das Instrument und einem am distalen Ende (3) ausgebildeten Nadelkopf (4) mit einer Wirkfläche (5) zum Abstrahlen von Ultraschallwellen, wobei sich in Axialrichtung ein Absaugkanal (6) zum Absaugen von Linsentrümmern durch die Hohlnadel hindurch erstreckt, dessen Öffnung (7) durch die Wirkfläche (5) begrenzt ist, wobei der Absaugkanal (6) im Nadelkopf (4) zumindest bereichsweise in Richtung des proximalen Endes (1) konisch zulaufend ausgebildet ist und wobei über diesen Bereich hinweg in Axialrichtung verlaufende Einschnitte (9) in der Wandung (10) des Absaugkanals (6) ausgebildet sind
**dadurch gekennzeichnet, dass** die Wirkfläche (5) zwei in unterschiedlichen Winkeln gegenüber der Axialrichtung verlaufende Wirkflächenbereiche (13, 14) aufweist, so dass die Wirkfläche (15) schnabelartig ausgebildet ist.

2. Hohlnadel nach Anspruch 1, **dadurch gekennzeichnet, dass** unmittelbar am distalen Ende des Nadelkopfes (4) in der Wandung (10) des Absaugkanals (6), insbesondere tetraederförmige, Ausnehmungen ausgebildet sind.

3. Hohlnadel nach Anspruch 1 oder 2, **dadurch gekennzeichnet, dass** die Einschnitte (9) einen mehreckigen, insbesondere dreieckigen, Querschnitt aufweisen.

4. Hohlnadel nach einem der Ansprüche 1 bis 3, **dadurch gekennzeichnet, dass** die Einschnitte (9) in Umfangsrichtung radialsymmetrisch oder asymmetrisch angeordnet sind.

5. Hohlnadel nach einem der Ansprüche 1 bis 4, **dadurch gekennzeichnet, dass** sich der konisch zulaufende Bereich (8) durch einen Teil des Nadelkopfes (4) oder durch den gesamten Nadelkopf (4) hindurch erstreckt.

6. Hohlnadel nach einem der Ansprüche 1 bis 5, **dadurch gekennzeichnet, dass** der konisch zulaufende Bereich (8) unmittelbar am distalen Ende (3) oder versetzt vom distalen Ende (3) - in Richtung des proximalen Endes (1) - beginnt.

7. Hohlnadel nach einem der Ansprüche 1 bis 6, **dadurch gekennzeichnet, dass** die Einschnitte (9) in Richtung des proximalen Endes (1) über den konisch zulaufenden Bereich (8) hinaus verlaufen.

8. Hohlnadel nach einem der Ansprüche 1 bis 7, **dadurch gekennzeichnet, dass** die äußere Wandung (11) des Nadelkopfes (4) rund oder vieleckig, insbesondere vier-, fünf-, sechs- oder achteckig, ausgebildet ist.

9. Hohlnadel nach einem der Ansprüche 1 bis 8, **dadurch gekennzeichnet, dass** die Wirkfläche (5) senkrecht oder schräg zu der Axialrichtung verläuft.

10. Hohlnadel nach einem der Ansprüche 1 bis 9, **dadurch gekennzeichnet, dass** die Wirkfläche (5) durch eine äußere Kante begrenzt ist, wobei die äußere Kante angefast, stumpf oder scharf ausgebildet ist.

## Claims

1. Hollow needle for an ophthalmic surgical instrument for in-vivo fragmentation of organic lenses by means of ultrasound, having a connection region (2) which is formed at the proximal end (1) for coupling to the instrument and a needle head (4) which is formed at the distal end (3) and which has an active face (5) for radiating ultrasound waves, wherein a suction channel (6) for drawing in lens debris extends in an axial direction through the hollow needle whose opening (7) is delimited by the active face (5), wherein the suction channel (6) is constructed so as to taper conically in the needle head (4) at least partially in the direction of the proximal end (1) and wherein incisions (9) which extend over this region in an axial direction are formed in the wall (10) of the suction channel (6), **characterised in that** the active face (5) has two active face regions (13, 14) which extend at different angles with respect to the axial direction so that the active face (15) is constructed in a beak-like manner.

2. Hollow needle according to claim 1, **characterised in that** directly at the distal end of the needle head (4) in the wall (10) of the suction channel (6) recesses, in particular tetrahedral recesses, are formed.

3. Hollow needle according to claim 1 or 2, **characterised in that** the incisions (9) have a polygonal, in particular triangular, cross-section.

4. Hollow needle according to any one of claims 1 to 3, **characterised in that** the incisions (9) are arranged in a peripheral direction in a radially symmetrical manner or asymmetrical manner.

5. Hollow needle according to any one of claims 1 to 4, **characterised in that** the conically tapering region (8) extends through a portion of the needle head (4) or through the entire needle head (4).

6. Hollow needle according to any one of claims 1 to 5, **characterised in that** the conically tapering region (8) begins directly at the distal end (3) or offset from the distal end (3) - in the direction of the proximal end (1).

7. Hollow needle according to any one of claims 1 to 6, **characterised in that** the incisions (9) extend in the direction of the proximal end (1) beyond the conically tapering region (8).

8. Hollow needle according to any one of claims 1 to 7, **characterised in that** the outer wall (11) of the needle head (4) is constructed in a round or polygonal, in particular pentagonal, hexagonal or octagonal, manner.

9. Hollow needle according to any one of claims 1 to 8, **characterised in that** the active face (5) extends perpendicularly or obliquely with respect to the axial direction.

10. Hollow needle according to any one of claims 1 to 9, **characterised in that** the active face (5) is delimited by an outer edge, wherein the outer edge is constructed to be chamfered, blunt or sharp.

## Revendications

1. Aiguille creuse pour un instrument de chirurgie ophtalmique pour la fragmentation in vivo de lentilles organiques au moyen d'ultrasons, avec une zone de raccordement (2) réalisée au niveau de l'extrémité proximale (1), pour le couplage à l'instrument et une tête d'aiguille (4) réalisée au niveau de l'extrémité distale (3), avec une surface active (5) pour l'émission d'ondes ultrasonores, un canal d'aspiration (6) pour l'aspiration de fragments de lentilles s'étendant dans la direction axiale à travers l'aiguille creuse, dont l'ouverture (7) est délimitée par la surface active (5), le canal d'aspiration (6) étant réalisée de manière conique au moins à certains endroits, en direction de l'extrémité proximale (1) et des entailles (9) s'étendant sur toute cette zone étant réalisées dans la paroi (10) du canal d'aspiration (6),
**caractérisée en ce que** la surface active (5) comprend deux zones de surface active (13, 14) s'étendant avec des angles différents par rapport à la direction axiale, de façon à ce que la surface active (15) soit réalisée sous la forme d'un bec.

2. Aiguille creuse selon la revendication 1, **caractérisée en ce que**, directement sur l'extrémité distale de la tête d'aiguille (4), dans la paroi (10) du canal d'aspiration (6) sont réalisées des évidements, plus particulièrement de forme tétraédrique.

3. Aiguille creuse selon la revendication 1 ou 2, **caractérisée en ce que** les entailles (9) présentent une section transversale polygonale, de préférence triangulaire.

4. Aiguille creuse selon l'une des revendications 1 à 3, **caractérisée en ce que** les entailles (9) sont disposées avec une symétrie radialement ou de manière asymétrique sur la circonférence.

5. Aiguille creuse selon l'une des revendications 1 à 4, **caractérisée en ce que** la zone conique (8) s'étend à travers une partie de la tête d'aiguille (4) ou à travers toute la tête d'aiguille (4).

6. Aiguille creuse selon l'une des revendications 1 à 5, **caractérisée en ce que** la zone conique (8) commence directement au niveau de l'extrémité distale (3) ou de manière décalée par rapport à l'extrémité distale (3), en direction de l'extrémité proximale (1).

7. Aiguille creuse selon l'une des revendications 1 à 6, **caractérisée en ce que** les entailles (9) s'étendent en direction de l'extrémité proximale (1) au-delà de la zone conique (8).

8. Aiguille creuse selon l'une des revendications 1 à 7, **caractérisée en ce que** la paroi externe (11) de la tête d'aiguille (4) présente une forme ronde ou polygonale, plus particulièrement quadrangulaire, pentagonale, hexagonale ou octogonale.

9. Aiguille creuse selon l'une des revendications 1 à 8, **caractérisée en ce que** la surface active (5) est perpendiculaire ou oblique par rapport à la direction axiale.

10. Aiguille creuse selon l'une des revendications 1 à 9, **caractérisée en ce que** la surface active (5) est délimitée par une arête externe, l'arête externe étant chanfreinée, émoussée ou affûtée.
